# EUROPEAN PATENT APPLICATION

(11) **EP 3 128 004 A1**
(43) Date of publication of application: **08.02.2017**
(21) Application number: 14887742.6
(22) Date of filing: 31.03.2014
(51) Int. Cl.: C12N 9/02, C12N 9/96

(54) **ENZYME AGENT**

(71) Applicant: Kewpie Corporation, Tokyo 150-0002 (JP)
(72) Inventor: ODAHARA, Makoto, Sashima-gun Ibaraki 306-0315 (JP); SEITO, Yayoi, Sashima-gun Ibaraki 306-0315 (JP); OKUYAMA, Yohei, Chofu-shi Tokyo 182-0002 (JP)
(74) Representative: Duckworth, Timothy John
(86) International application number: PCT/JP2014/059593
(87) International publication number: WO 2015/151216

(57) **Abstract**

There is provided an enzyme preparation capable of effectively removing C1-C10 aldehydes.

The enzyme preparation includes as an active ingredient a composite of aldehyde dehydrogenase produced by acetic acid bacteria of the genus Gluconacetobacter and cell membranes with which the aldehyde dehydrogenase combines. The enzyme preparation has a pH of 4 to 7. An activity of the aldehyde dehydrogenase per 0.006 mg of protein obtainable from the acetic acid bacteria is represented as absorbance (660 nm) of an aqueous solution (3.9 mL) of ferric ferrocyanide, the absorbance being 0.05 to 3, when the ferric ferrocyanide is generated from potassium ferricyanide along with oxidation of acetaldehyde by the enzyme preparation.

## Description

### Technical Field

The present invention relates to an enzyme preparation that includes, as an active ingredient for an enzyme activity, a composite of aldehyde dehydrogenase produced from cell membranes of acetic acid bacteria and cell membranes with which the aldehyde dehydrogenase combines, and to a method of producing the enzyme preparation.

### Background Art

C1-C10 aldehydes have high oxidation power and are harmful to living bodies. For example, it is a known fact that C1-C10 aldehydes become a cause of lifestyle-related diseases or cancer or accelerate cell aging. For example, formaldehyde has acute toxicity that irritates mucous membranes. Acetaldehyde causes a sickness from drinking and also becomes a cause of esophageal cancer.

Further, many aldehydes have particular odors. For example, octanal and nonenal are perceived as unpleasant odor components emitted from the body surfaces of humans. Acetaldehyde is perceived as an odor emitted from a mouth or exhaled breath in drinking or smoking. Hexanal and heptanal are perceived as deterioration odor components emitted from foods such as rice and soybeans.

Harmful effects of the above-mentioned aldehydes to living bodies have been raised for the last 50 years. In particular, people nowadays have problems in overconsumption of carbohydrates and lipids and in daily stress, and thus reduction of C1-C10 aldehydes existing in or out of human bodies is an important issue.

A conventionally-known agent for removing aldehyde is one produced by fixing an enzyme, which is derived from Aspergillus oryzae incorporated with an alcohol oxidase gene, on a carrier having a surface modified with an amino group (Patent Document 1).

However, the effectiveness of the agent for removing aldehyde is limited to C1 formaldehyde in C1-C10 aldehydes.

Further, it is known that in order to reduce medium-chain aldehydes, aldehyde dehydrogenase of acetic acid bacteria selected from the genus Gluconobacter and the genus Acetobacter is put to practical use (Patent Documents 2 and 3).

However, when the inventors of the present invention verified those examples by using Acetobacter pasteurianus (IFO3283) and Gluconobacter oxidans (IFO12528), an odor derived from aldehyde or cultures was perceived, and an effect of reducing an aldehyde odor was found insufficient.

Patent Document 1: Japanese Patent No. 4374595
Patent Document 2: Japanese Patent No. 2521460
Patent Document 3: Japanese Examined Patent Application Publication No. Hei 6-97959

### Summary of Invention

### Problem to be solved by the Invention

In contrast to the above-mentioned technology in the related art, it is an object of the present invention to provide an enzyme preparation capable of effectively removing C1-C10 aldehydes.

### Means for solving the Problem

The inventors of the present invention found the following fact: when acetic acid bacteria of the genus Gluconacetobacter having a high acetic acid resistance in acetic acid bacteria used for producing acetic acid are cultured in the presence of ethanol, and when the culture is stopped before an acetic acid acidity reaches 4% or more, aldehyde dehydrogenase combining with cell membranes of the acetic acid bacteria exerts a high aldehyde dehydrogenase activity. Thus, the inventors have conceived the present invention.

Specifically, according to the present invention, there is provided an enzyme preparation, including as an active ingredient a composite of aldehyde dehydrogenase produced by acetic acid bacteria of the genus Gluconacetobacter and cell membranes with which the aldehyde dehydrogenase combines, the enzyme preparation having a pH of 4 to 7, an activity of the aldehyde dehydrogenase per 0.006 mg of protein obtainable from the acetic acid bacteria being represented as absorbance (660 nm) of an aqueous solution (3.9 mL) of ferric ferrocyanide, the absorbance being 0.05 to 3, when the ferric ferrocyanide is generated from potassium ferricyanide along with oxidation of acetaldehyde by the enzyme preparation.

Further, according to the present invention, there is provided a method of producing the enzyme preparation described above, the method including culturing the acetic acid bacteria of the Gluconacetobacter in a medium including carbohydrates, vitamins, minerals, and ethanol at an acetic acid acidity of less than 4%, to include as an active ingredient a composite of aldehyde dehydrogenase of the acetic acid bacteria and cell membranes in the culture solution.

### Effects of the Invention

With the enzyme preparation of the present invention, it is possible to effectively remove C1-C10 aldehydes. Further, a composite of aldehyde dehydrogenase from acetic acid bacteria and cell membranes is conventionally used for production of vinegar and can thus be used safely.

For that reason, using the enzyme preparation of the present invention for oral agents, mouth cleaning agents, external agents, cosmetics, food additive agents, deodorizers for household use, and the like enables reduction of aldehydes harmful to living bodies, and also enables reduction of bad odors due to aldehydes.

### Brief Description of Drawings

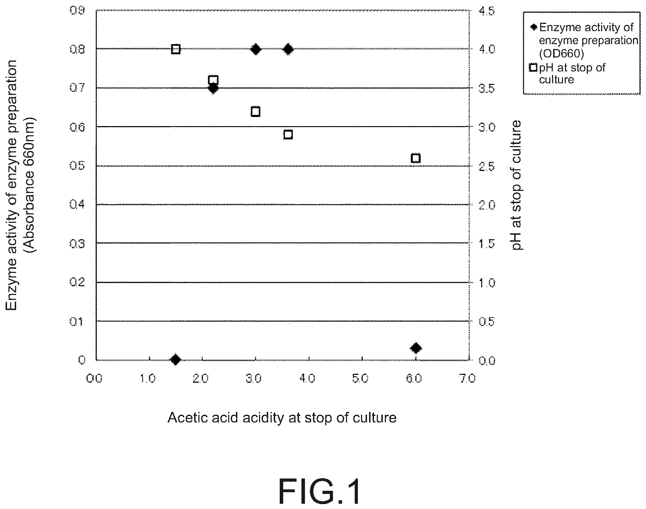

[Fig. 1] Fig. 1 is a relationship diagram of an acetic acid acidity when culture of acetic acid bacteria is stopped, and an aldehyde dehydrogenase activity of a culture solution of the acetic acid bacteria, in Examples 1 to 3 and Comparative Examples 1 and 2.

### Mode(s) for Carrying Out the Invention

Hereinafter, the present invention will be described in detail. It should be noted that in the present invention, unless otherwise defined, part(s) represent(s) mass parts and % represents mass%.

### <Acetic Acid Bacteria>

An enzyme preparation of the present invention includes, as an active ingredient, a composite of aldehyde dehydrogenase and cell membranes with which the aldehyde dehydrogenase combines. The aldehyde dehydrogenase is obtained by culturing acetic acid bacteria of the genus Gluconacetobacter under specific conditions.

Acetic acid bacteria refer to a generic name of microorganisms that grow while using sugars or sugar alcohols and oxidize ethanol, to generate acetic acid. Examples of representative types of acetic acid bacteria used for production of fermented vinegar include the genus Acetobacter, the genus Gluconobacter, and the genus Gluconacetobacter. Of those, the genus Gluconacetobacter includes strains having a high acetic acid resistance. With use of the acetic acid bacteria of the genus Gluconacetobacter, transformed strains having a higher acetic acid resistance are obtained (see Japanese Patent No. 4326967).

Because of having a high acetic acid resistance, the acetic acid bacteria of the genus Gluconacetobacter are used in the present invention.

Examples of bacteria of the genus Gluconacetobacter include Gluconacetobacter diazotrophicus, Gluconacetobacter azotocaptans, Gluconacetobacter swingsii, Gluconacetobacter xylinus, Gluconacetobacter europaeus, Gluconacetobacter maltaceti, Gluconacetobacter kombuchae, Gluconacetobacter hansenii, and Gluconacetobacter liquefaciens. Of those, Gluconacetobacter swingsii, Gluconacetobacter xylinus, Gluconacetobacter europaeus, Gluconacetobacter maltaceti, and the like can provide a high aldehyde dehydrogenase activity. One or a plurality of types of them may be used.

Normally, when acetic acid bacteria are cultured, an acetic acid acidity in a medium rises along with the proliferation of the acetic acid bacteria. When the acetic acid acidity in the medium reaches 6% or more, almost all of the acetic acid bacteria in such a medium are killed within 12 hours. In contrast to this, the present invention preferably uses acetic acid bacteria capable of growing at the acetic acid acidity of 6% or more, and thus uses acetic acid bacteria of the genus Gluconacetobacter. Here, "capable of growing at the acetic acid acidity of 6% or more" means that acetic acid bacteria can proliferously grow while producing acetic acid even at the acetic acid acidity of 6% or more.

As described above, the acetic acid bacteria having a high acetic acid resistance are used. This is because the inventors of the present invention obtained the following knowledge: when culture of acetic acid bacteria having an acetic acid resistance is started in a medium including at least carbohydrates, vitamins, minerals, and ethanol, particularly in a medium having an ethanol concentration of 3% or more, and when the culture is stopped at the time the acetic acid acidity is less than 4%, particularly, the acetic acid acidity reaches 2 to 3.9%, an activity per unit weight of the aldehyde dehydrogenase is significantly increased, that is, the number of active enzymes is significantly increased.

It should be noted that regarding the acetic acid resistance of acetic acid bacteria, even when the upper limit of the acetic acid acidity at which the acetic acid bacteria are capable of growing exceeds 10%, the aldehyde dehydrogenase activity of the acetic acid bacteria is almost the same as an aldehyde dehydrogenase activity when the upper limit is 6%.

### <pH>

The enzyme preparation of the present invention is adjusted to have a pH of 4 to 7, particularly, a pH of 5 to 6.5 by using a pH buffer solution including citric acid or the like.

When the enzyme preparation is liquid, the pH value described above can be directly measured with a pH meter. When the enzyme preparation is solid such as powder, the pH value can be measured by dispersing the enzyme preparation into distilled water such that a solid content concentration is 1 to 10%.

Maintaining the pH of the enzyme preparation in a range of 4 to 7 enables the aldehyde dehydrogenase activity in the enzyme preparation to be maintained for a long time. For example, even after the elapse of one month from the formulation, the aldehyde dehydrogenase activity can be maintained to be 70% or more of the original aldehyde dehydrogenase activity.

### <Acquisition of Acetic Acid Bacteria>

The acetic acid bacteria used in the present invention can be acquired by screening, from acetic acid bacteria of the genus Gluconacetobacter conventionally used for production of fermented vinegar, acetic acid bacteria having a high acetic acid resistance, i.e., acetic acid bacteria capable of growing at the acetic acid acidity of 5% or more, particularly, at the acetic acid acidity of 6% or more.

Further, for the acetic acid bacteria capable of growing at the acetic acid acidity of 5% or more, acetic acid bacteria of the genus Gluconacetobacter that are transformed by a method described in Japanese Patent No. 4326967 may be used.

### <Method of Producing Enzyme Preparation>

The enzyme preparation of the present invention can be produced as described below by using the acetic acid bacteria having a high acetic acid resistance, preferably the acetic acid bacteria capable of growing at the acetic acid acidity of 6% or more. Such acetic acid bacteria are acquired by screening from the acetic acid bacteria of the genus Gluconacetobacter conventionally used for production of fermented vinegar.

### <Culture of Acetic Acid Bacteria>

The acetic acid bacteria of the genus Gluconacetobacter are cultured in a medium including at least carbohydrates obtainable from glucose and the like, vitamins and minerals obtainable from yeast extracts and the like, and ethanol. In this case, the acetic acid bacteria of the genus Gluconacetobacter are preferably cultured under culture conditions of an ethanol concentration of 3% or more in the medium, an initial temperature of 25 to 35°C, and the amount of ventilation of 0.02 to 5 L/min per liter of the medium. The culture is performed at the acetic acid acidity of less than 4% in the culture solution. Specifically, along with the proliferation of the acetic acid bacteria in the medium, the ethanol in the medium is oxidized to form aldehyde and then acetic acid. The acetic acid acidity in the medium rises, but in the present invention, the culture is stopped before the acetic acid acidity reaches 4%, preferably when the acetic acid acidity is 2 to 3.9%, particularly 2.5 to 3.5%.

It should be noted that according to Japanese Agricultural Standards (JAS), fermented vinegar has an acetic acid concentration of 4% or more, and vinegar used as seasoning at home and the like has an acetic acid concentration of 4 to 5%. Thus, when vinegar is generally produced, the culture of acetic acid bacteria is continued until acetic acid acidity in a culture solution reaches 4% or more. In particular, in terms of increase in production efficiency of the fermented vinegar, the culture is continued until the acetic acid acidity becomes as high as possible and higher than 4%, the culture solution with an increased acetic acid acidity is diluted with freshwater, and vinegar is thus produced (Japanese Patent No. 3437324).

In contrast to this, the present invention is different from a conventional method of culturing acetic acid bacteria in that even when the acetic acid bacteria of the genus Gluconacetobacter are capable of growing at the acetic acid acidity of 6% or more, the culture is stopped before the acetic acid acidity of the culture solution reaches 4%, as described above.

In order to stop the culture, it is preferable to stop ventilation and shut off supply of oxygen, because the acetic acid bacteria are aerobic microorganisms.

In the acetic acid bacteria cultured as described above, the aldehyde dehydrogenase combines with cell membranes of the acetic acid bacteria to form a composite. The formation of a composite can be visually recognized with an electron microscope, for example.

### <Aldehyde Dehydrogenase Activity>

The enzyme preparation of the present invention includes a composite of aldehyde dehydrogenase and cell membranes. An aldehyde dehydrogenase activity of the composite per 0.006 mg of protein obtainable from acetic acid bacteria is represented as absorbance (660 nm) of an aqueous solution (3.9 mL) of ferric ferrocyanide when the ferric ferrocyanide is generated from potassium ferricyanide along with the oxidation of acetaldehyde. The absorbance is 0.05 to 3. More specifically, the aldehyde dehydrogenase activity can be measured as follows according to a method of using ferricyanide for electron acceptors by Wood et al. (Methods in Enzymology, Volume 5, 1962, Pages 287-291).

Specifically, after the culture is stopped, the culture solution is subjected to centrifugation, and the concentration of the culture solution is adjusted using a McIlvaine buffer (pH5), to prepare a dispersion liquid of acetic acid bacteria of 0.1 mg/mL. 0.1 mL of the dispersion liquid of acetic acid bacteria (0.01 mg as acetic acid bacteria cells) and 0.9 mL of an aqueous solution including 0.02 mmol of acetaldehyde and 0.01 mmol of potassium ferricyanide are held at 37°C for 20 minutes. Thus, aldehyde dehydrogenase in the dispersion liquid of acetic acid bacteria is reacted with the aldehyde, to oxidize the aldehyde. As a side reaction of the oxidation, the potassium ferricyanide is reduced to ferric ferrocyanide (Prussian blue). This reaction is stopped by adding 0.5 mL of a Dupanol reagent. 2.5 mL of distilled water is further added thereto, and absorbance at 660 nm of the ferric ferrocyanide (Prussian blue) is measured. In this case, for a blank test, a sample including no dispersion liquid of acetic acid bacteria is measured.

In such a manner, the amount of generation of Prussian blue per 0.01 mg of acetic acid bacteria, which is measured as absorbance at 660 nm, i.e., the amount of generation of Prussian blue per 0.006 mg of protein obtainable from acetic acid bacteria because protein content of the acetic acid bacteria is 60%, corresponds to the amount of aldehyde oxidized by the aldehyde dehydrogenase. In the present invention, the thus-measured absorbance (660 nm) per 0.006 mg of protein obtainable from acetic acid bacteria is 0.05 to 3 as described above. This value has not been achieved by a conventional composite of aldehyde dehydrogenase and cell membranes. The composite used in the present invention is different from the conventional composite in that the number of active enzymes per unit weight of the protein obtainable from acetic acid bacteria is significantly increased.

It should be noted that the acetic acid bacteria include various types of proteins, and proteins constituting the aldehyde dehydrogenase are found in bands of 86kD and 55kD. The presence of those proteins can be confirmed by subjecting those proteins to Polyacrylamide gel electrophoresis (SDS-PAGE) using 12.5% Polyacrylamide gels.

### <Formulation>

The culture solution, in which the culture is stopped when the acetic acid acidity is less than 4% as described above, includes a composite of aldehyde dehydrogenase that combines with bacterial membranes. Further, the culture solution includes acetic acids, amino acids, other organic acids, peptides, produced polysaccharides such as cellulose, Maillard reactants such as melanoidin, and the like. In general, in fermented foods such as miso and soy sauce, odors of the foods are suppressed by using a complicated flavor generated by culture. In the present invention, however, in terms of a high temporal stability of the aldehyde dehydrogenase activity, the composite of aldehyde dehydrogenase and bacterial membranes is preferably used separately from fermentation components such as the acetic acid described above. In this regard, first, the culture solution is subjected to centrifugal treatment (3000 to 15000 rpm), membrane concentration treatment, drying treatment, and the like, to concentrate the acetic acid bacteria cells to 1 to 100%. The acetic acid bacteria cells include the aldehyde dehydrogenase in the state of combining with the cell membranes. Next, in order to further remove medium components from the concentrated culture solution, washing may be performed. A washing method is not particularly limited. For example, medium components are removed by diluting and mixing the culture solution concentrated to 10% acetic acid bacteria cells with a buffer solution including citric acid or phosphoric acid, performing centrifugal treatment thereon, and removing a supernatant thereof.

After the concentration and, as needed, the removal of the fermentation components such as acetic acid, a pH buffer solution is used to adjust a pH to 4 to 7, particularly 5 to 6.5. Thus, an enzyme preparation in any form such as powder, granules, capsules, liquid, and cream can be formed by an ordinary method. In such a manner, when the pH is adjusted with a buffer solution and the culture solution is then formulated into powder or the like, the pH does not vary even when powder absorbs water from the air, so that a pH with a stable enzyme activity can be maintained. For the buffer solution used to adjust the pH, citric acid is preferable in terms of having non-volatility and a good temporal stability.

Specific formulation methods are as follows, for example: for powder, the acetic acid bacteria cells are mixed with dextrin, lactose, and the like and subjected to spray drying treatment or lyophilization treatment, to be dried while thermal damage is being suppressed; for capsules, the acetic acid bacteria cells are subjected to coating treatment using gelatin or guar gum to be shaped; and for liquid, since the composite of aldehyde dehydrogenase and bacterial membranes is water-insoluble, starch or the like is used to increase the viscosity to 1 Pa·s or more, and the acetic acid bacteria cells are dispersed in the liquid.

It should be noted that when protein obtainable from milk, protein obtainable from soybeans, or the like is used as an excipient in the formulation, the protein used as an excipient and the protein obtainable from acetic acid bacteria can be distinguished from each other by electrophoretic patterns when all the proteins are subjected to Polyacrylamide gel electrophoresis (SDS-PAGE) using 12.5% Polyacrylamide gels.

Further, while the aldehyde dehydrogenase needs coenzyme so as to express the enzyme activity thereof, the coenzyme also combines with the cell membranes. Thus, when the formulation is performed with the aldehyde dehydrogenase combining with the cell membranes, the enzyme activity can be obtained also after the formulation.

### <Use Application of Enzyme Preparation>

The enzyme preparation of the present invention has an effect of removing C1-C10 aldehydes. Here, examples of C1-C10 aldehydes include formaldehyde, acetaldehyde, butanal, glyceraldehyde, malondialdehyde, hexanal, heptanal, octanal, nonenal, decanal, isooctanal, isononenal, and isodecanal.

The enzyme preparation of the present invention can be used in any situation where aldehydes are intended to be reduced. For example, in order to reduce aldehydes in living bodies, the enzyme preparation of the present invention may be orally ingested or may be applied as an external agent. Aldehydes become a cause of oxidative stress on human bodies or an odor when the concentration at volatilization is only 0.1 to several tens of ppm. For use of the enzyme preparation of the present invention through oral ingestion or application, the amount of usage thereof may be set to any amount with which aldehydes can be reduced. For example, the amount of usage of the enzyme preparation may be 1 to 100 mg per one food by oral ingestion or 100 cm² by application.

Further, in order to reduce or eliminate environmental aldehydes such as odors from wall materials of buildings and in public facilities where people gather, the enzyme preparation of the present invention may be applied or sprayed to the wall materials and the like.

For foods, the enzyme preparation of the present invention can be used in combination with the following ingredients without impairing the effects of the enzyme preparation of the present invention. Examples of the ingredients includes: sweeteners such as trehalose, sucrose, aspartame, and phenylalanine; acidulants such as citric acid, acetic acid, lactic acid, phosphoric acid, and lemon juice; other seasonings such as sucrose, lactose, salt, and soy sauce; crude drugs such as turmeric, liver extract, cinnamon, fennel, peony, and licorice; beauty materials such as collagen, hyaluronic acid, placenta, coenzyme Q10, chondroitin, and royal jelly; amino acids such as taurine and alanine; polysaccharide thickeners such as soybean polysaccharides, guar gum, and xanthan gum; and flavoring agents such as grapefruit and lemon.

For cosmetics, the following ingredients can be used in combination with the enzyme preparation of the present invention: porous particulates such as cyclodextrin, diatomaceous earth, and activated charcoal; polysaccharides such as crystalline cellulose, carboxymethyl cellulose, and dextrin; botanical extracts such as polyphenol, Eucalyptus extract, Tea catechin, and Oenothera biennis extract; bactericidal agents or preservatives such as isopropyl methyl phenol, triclosan, benzoic acid, benzylglyceryl ether, silver compound, methylparaben, ethylparaben, butylparaben, propylparaben, phenoxyethanol, hydroxamic acid derivatives, sophora flavescens extract, alkyl diethanol, and ethanol; silicon oils such as polysiloxane, talc, and cyclic siloxane; oils and fats such as isopropyl myristate, squalane, and paraffin; anti-inflammatory agents such as glycerrhizinic acid, urea, Scutellaria baicalensis extract, and Phellodendron amurense extract; astringents such as
zinc oxide, magnesium oxide, and aluminum oxide; beauty ingredients such as hyaluronic acid, collagen, coenzyme Q10, chondroitin, placenta, ceramide, elastin, astaxanthin, royal jelly, aloe extract, platinum nano colloid, retinoic acid, retinol, hydrolyzed wheat, Psophocarpus tetragonolobus extract, lactoferrin, soymilk fermentation liquor, and epidermal growth factor (EGF); skin-whitening ingredients such as arbutin, fullerene, tranexamic acid, hydroquinone, and Coix seed extract; ultraviolet absorbers such as benzophenone derivatives, p-aminobenzoic acid derivatives, and methoxy cinnamic acid derivatives; humectants such as pentylene glycol, hexanediol, caprylyl glycol, glycol, propylene glycol, butylene glycol, polyethylene glycol, and adenosine; anionic surfactants such as alkylbenzene sulfonate, polyoxyalkylene alkyl ether sulfate, alkyl sulfate, olefin sulfonate, fatty acid salt, and dialkyl sulfosuccinate salt; nonionic surfactants such as polyethylene glycol hydrogenated castor oil, polyoxyethylene fatty acid ester, and polyoxyethylene hydrogenated castor oil derivatives; cationic surfactants such as alkyltrimethylammonium salts, dialkyldimethylammonium salts, alkylpyridinium salts, and stearyl trimethyl ammonium chloride; amphoteric surfactants such as alkyl betaines, alkyl amidopropyl betaines, imidazolinium betaines, egg yolk lecithin, and soybean lecithin; various amino acids; various vitamins; various peptides; higher fatty acids; polyhydric alcohols; thickening agents; carbonates; emulsifiers; and perfumes.

### [Examples]

Hereinafter, the present invention will be described in detail on the basis of Examples.

### Example 1

0.01 part of acetic acid bacteria strains (Gluconacetobacter swingsii QPJ strains) was added to 0.5 parts of glucose, 0.5 parts of yeast extract, 4.8 parts of ethanol, 1.5 parts of acetic acid, and 92.7 parts of freshwater, and culture was started under conditions of an initial temperature of 30°C and the amount of ventilation of 0.1 L/min per liter of the medium. The Gluconacetobacter swingsii QPJ strains were obtained by screening strains capable of growing at the acetic acid acidity of 6% or more from Gluconacetobacter swingsii used for production of fermented vinegar.

After 18 hours from the start of the culture, the ventilation was stopped and the culture was also stopped at the timing the acetic acid acidity reached 2.2%, to obtain a culture solution of acetic acid bacteria. The obtained culture solution of acetic acid bacteria was subjected to centrifugal treatment at 10000 rpm to be concentrated to 10% solid content, and was then sufficiently mixed using a citrate buffer solution (pH5) having a volume ten times as large as the concentrate in terms of mass. The centrifugal treatment at 10000 rpm was performed again to remove a supernatant, so that culture-solution components were removed. The concentration was adjusted using a citrate buffer solution (pH5), to obtain a liquid enzyme preparation (including 6% protein obtainable from acetic acid bacteria) (including a cell membrane composite of aldehyde dehydrogenase).

### <Deodorant Test>

A conical flask having a 300 mL volume and provided with filter paper therein was prepared. On the filter paper, 1 µL of acetaldehyde and 1 mL of the liquid enzyme preparation of Example 1 were dropped and sealed with a lid. At that time, the concentration of acetaldehyde in a headspace of the conical flask was 100 ppm, which was measured with a gas detector tube. As a result of heat retaining treatment at 37°C for 30 minutes, an acetaldehyde odor disappeared, and the concentration of aldehyde in the air of the headspace of the conical flask was lowered to 0.2 ppm or less, which was measured with the gas detector tube.

Instead of acetaldehyde, nonenal was used for the deodorant test in a similar manner described above. As a result, after the heat retaining treatment, a nonenal odor disappeared, and the concentration of nonenal was lowered to 0.2 ppm or less, which was measured with the gas detector tube.

Instead of 1 mL of the liquid enzyme preparation of Example 1, water was used for the deodorant test in a similar manner. An acetaldehyde odor and a nonenal odor did not disappear, and particular unpleasant odors emanated. Further, the concentration of acetaldehyde was 100 ppm, which was measured with the gas detector tube.

### <Aldehyde Dehydrogenase Activity>

The aldehyde dehydrogenase activity of the liquid enzyme preparation of Example 1 was measured as follows.

0.125 mL of a McIlvaine buffer solution (pH5), 0.2 mL of 0.1 M acetaldehyde, and 0.1 mL of the liquid enzyme preparation of Example 1 (0.1 mg/mL of acetic acid bacteria) were mixed and adjusted to have the total volume of 0.8 mL with use of distilled water. 0.1 mL of 0.1 M potassium ferricyanide was added thereto, heat retaining treatment was performed thereon at 37°C for 20 minutes, and an enzymatic reaction was stopped by adding 0.5 mL of a Dupanol reagent thereto. Next, the resultant solution was mixed with 2.5 mL of distilled water and left at room temperature for 20 minutes. Subsequently, coloring of the produced Prussian blue was measured at absorbance of 660 nm. In this case, for a blank test, a sample including no dispersion liquid of acetic acid bacteria was measured.

As a result, when the aldehyde dehydrogenase activity was measured for 0.01 mL of the liquid enzyme preparation of Example 1, the absorbance (660 nm) of the Prussian blue produced in parallel with the oxidation of aldehyde was 0.7.

Further, after the liquid enzyme preparation of Example 1 was preserved at 37°C for 7 days, the aldehyde dehydrogenase activity was similarly measured. The absorbance (660 nm) of the Prussian blue was 0.6.

### <Upper Limit Acidity for Growth of Acetic Acid Bacteria>

Acetic acid bacteria were cultured similarly except that an ethanol concentration at the start of culture was increased. When the presence or absence of the growth of acetic acid bacteria at a high acetic acid acidity was checked, it was found that the acetic acid bacteria could grow at the acetic acid acidity of up to 8% or more.

### Examples 2 and 3 and Comparative Examples 1 and 2

In Example 1, acetic acid bacteria were cultured similarly to Example 1 except that the acetic acid acidity at the stop of culture was set as shown in Table 1. A deodorant test was performed using the obtained liquid enzyme preparation, and the aldehyde dehydrogenase activity was measured.

Here, a culture solution of acetic acid of Comparative Example 1 is the culture solution of acetic acid at the start of culture.

Results of the deodorant tests and measurement results of the aldehyde dehydrogenase activity in Examples 1 to 3 and Comparative Examples 1 and 2 are shown in Table 1. Further, relationships between the acetic acid acidity and the absorbance or pH at the stop of culture in those Examples are shown in Fig. 1.

**[Table 1]**

| | Comparative Example 1 | Example 1 | Example 2 | Example 3 | Comparative Example 2 |
|---|---|---|---|---|---|
| Acetic acid acidity at stop of culture | 1.5 | 2.2 | 3.0 | 3.6 | 6.0 |
| pH at stop of culture | 4.0 | 3.6 | 3.2 | 2.9 | 2.6 |
| Enzyme activity of enzyme preparation (OD660) | 0 | 0.7 | 0.8 | 0.8 | 0.03 |
| Deodorant Test (presence or absence of aldehyde odor) | present | absent | absent | absent | present |

### Comparative Example 3

A liquid enzyme preparation (including 6% protein obtainable from acetic acid bacteria) was obtained according to Example 3 except that 0.1% Triton of a surfactant was blended when the concentration was adjusted with use of the citrate buffer solution (pH5) for the culture solution of acetic acid bacteria, in which the culture is stopped at the acetic acid acidity of 3.6% obtained in Example 3. As a result of storage of the liquid enzyme preparation at 37°C for 7 days, the absorbance (660 nm) of the Prussian blue was lowered to 0.008. In this enzyme preparation, an enzyme activity was obtained from a fraction of a supernatant when centrifugal treatment was performed, and it was thus found that enzymes were separated from the cell membranes.

### Comparative Example 4

A liquid enzyme preparation (including 6% protein obtainable from acetic acid bacteria) was obtained according to Example 3 except that the citrate buffer solution (pH5) was replaced with an acetic acid buffer solution (pH3) for the culture solution of acetic acid bacteria, in which the culture is stopped at the acetic acid acidity of 3.6% obtained in Example 3. As a result of storage of the liquid enzyme preparation at 37°C for 7 days, the absorbance (660 nm) of the Prussian blue was 0.06.

### Comparative Example 5

For the acetic acid bacteria, Gluconobacter oxidans (IFO3287) were used. When the acetic acid bacteria were cultured according to Example 1, and when the aldehyde dehydrogenase activity was measured for the culture solution of acetic acid bacteria, in which the culture was stopped at the acetic acid acidity of 3%, the absorbance (660 nm) of the Prussian blue was 0.002. Further, when a deodorant test was performed for the culture solution according to Example 1, odors particular to acetaldehyde remained.

Because of a low acetic acid resistance, the Gluconobacter oxidans could not grow at the acetic acid acidity of 6%.

### Example 4

Gluconacetobacter xylinum QPJ strains were used instead of the Gluconacetobacter swingsii QPJ strains of Example 1. The Gluconacetobacter xylinum QPJ strains were obtained by screening strains capable of growing at the acetic acid acidity of 6% or more from Gluconacetobacter xylinus used for production of fermented vinegar.

Similarly to Example 1 except for use of those strains and the stop of culture at the acetic acid acidity of 3.7%, a liquid enzyme preparation was obtained, and the aldehyde dehydrogenase activity of the liquid enzyme preparation was measured. The absorbance (660 nm) of the Prussian blue was 0.8. Further, when a deodorant test was performed for acetaldehyde and nonenal as in Example 1, an acetaldehyde odor and a nonenal odor disappeared.

### Example 5

Gluconacetobacter europaeus QPJ strains were used instead of the Gluconacetobacter swingsii QPJ strains of Example 1. The Gluconacetobacter europaeus QPJ strains were obtained by screening strains capable of growing at the acetic acid acidity of 6% or more from acetic acid bacteria of the genus Gluconacetobacter, which were used for production of fermented vinegar.

Similarly to Example 1 except for use of those strains and the stop of culture at the acetic acid acidity of 3.7%, a liquid enzyme preparation was obtained, and the aldehyde dehydrogenase activity of the liquid enzyme preparation was measured. The absorbance (660 nm) of the Prussian blue was 1.0. Further, when a deodorant test was performed for acetaldehyde and nonenal as in Example 1, an acetaldehyde odor and a nonenal odor disappeared.

### Example 6

Gluconacetobacter maltaceti (IFO14815) were used instead of the Gluconacetobacter swingsii QPJ strains of Example 1. The Gluconacetobacter maltaceti (IFO14815) were used for production of fermented vinegar and capable of growing at the acetic acid acidity of 6% or more.

Similarly to Example 1 except for use of those strains and the stop of culture at the acetic acid acidity of 3.7%, a liquid enzyme preparation was obtained, and the aldehyde dehydrogenase activity of the liquid enzyme preparation was measured. The absorbance (660 nm) of the Prussian blue was 0.3. Further, when a deodorant test was performed for acetaldehyde and nonenal as in Example 1, an acetaldehyde odor and a nonenal odor disappeared.

### Example 7 (Food)

The concentration was adjusted with dextrin and freshwater such that the liquid enzyme preparation obtained in Example 2 (including 6% protein obtainable from acetic acid bacteria) was 1%, dextrin (dry mass) was 9%, and freshwater was 90%. The resultant solution was subjected to lyophilization treatment, and a powdered enzyme preparation (including 6% protein obtainable from acetic acid bacteria) was prepared.

79.9 parts of glucose, 16 parts of gelatin, 3 parts of citric acid, 1 part of the powdered enzyme preparation (including 6% protein obtainable from acetic acid bacteria) described above, and 0.1 part of aspartame were mixed, to prepare tablets each having 4 g by an ordinary method. Eating of the obtained tablets enabled bad odors from mouths due to acetaldehyde to be improved.

### Example 8 (Deodorizing Spray)

90 parts of liquefied petroleum gas, 7.7 parts of cetyl 2-ethylhexanoate, 1 part of anhydrous silica, 1 part of hydrogenated castor oil, 0.2 parts of glycerin, and 0.1 part of a powdered enzyme preparation similar to the powdered enzyme preparation of Example 7 were mixed to prepare a spray agent contained in a spray can of 180 mL by an ordinary method. The obtained spray agent was sprayed and applied to cotton undershirts worn by fifty-something males for 24 hours, and bad odors due to nonenal were reduced.

### Example 9 (Wet Sheet-Like Cosmetics)

10 parts of ethanol, 2 parts of aluminum hydroxychloride, 0.1 part of a powdered enzyme preparation similar to the powdered enzyme preparation of Example 7, 1 part of talc, 0.1 part of hydrogenated castor oil, 0.05 parts of menthol, and 86.75 parts of freshwater were infiltrated into a 10-cm-square polyester sheet according to an ordinary method. When fifty-something males used the obtained wet sheet-like cosmetics to wipe their necks, bad odors due to nonenal were reduced.

### Example 10 (Skin Cleansing Agent)

55 parts of glycerin, 8 parts of dipropylene glycol, 8 parts of diglycerin (product name "Diglycerin 801", manufactured by Sakamoto Yakuhin kogyo Co., Ltd.), 2 parts of ethanol, 0.5 parts of hydrogenated castor oil, 0.5 parts of polyoxyethylene sorbitan monolaurate (product name "Tween L120", manufactured by Kao Corporation), 0.2% parts of perfume, 0.1 part of sucrose fatty acid ester, 0.1 part of citric acid, 0.1 part of sodium citrate, 0.1 part of potassium hydroxide, 0.1 part of phenoxyethanol, and 18.3 parts of freshwater were mixed. Subsequently, 5 parts of magnesium oxide, 0.1 part of a powdered enzyme preparation similar to the powdered enzyme preparation of Example 7, and 1 part of vinyl methicone were added and mixed. When fifty-something males used the obtained skin cleansing agent to cleanse their armpits, a good effect of reducing bad odors due to nonenal was found.

### Example 11 (Beverage)

0.1 part of a powdered enzyme preparation similar to the powdered enzyme preparation of Example 7, 0.05 parts of turmeric powder, 0.05 parts of alanine, 0.05 parts of liver extract, 0.05 parts of vitamin C, 0.05 parts of vitamin E, 0.005 parts of citric acid, 0.005 parts of acetic acid, 4 parts of sucrose, 0.05 parts of soybean dietary fiber, and 95.59 parts of freshwater were mixed, to prepare packaged beverages each having 100 g by an ordinary method. When the obtained packaged beverage was ingested after drinking of 800 mL of beer, a sickness from drinking due to acetaldehyde was alleviated.

## Claims

1. An enzyme preparation, comprising as an active ingredient a composite of aldehyde dehydrogenase produced by acetic acid bacteria of the genus Gluconacetobacter and cell membranes with which the aldehyde dehydrogenase combines,
the enzyme preparation having a pH of 4 to 7,
an activity of the aldehyde dehydrogenase per 0.006 mg of protein obtainable from the acetic acid bacteria being represented as absorbance (660 nm) of an aqueous solution (3.9 mL) of ferric ferrocyanide, the absorbance being 0.05 to 3, when the ferric ferrocyanide is generated from potassium ferricyanide along with oxidation of acetaldehyde by the enzyme preparation.

2. The enzyme preparation according to claim 1, wherein
the acetic acid bacteria belong to Gluconacetobacter swingsii.

3. The enzyme preparation according to claim 1, wherein
the acetic acid bacteria belong to Gluconacetobacter xylinus.

4. The enzyme preparation according to claim 1, wherein
the acetic acid bacteria belong to Gluconacetobacter europaeus.

5. The enzyme preparation according to claim 1, wherein
the acetic acid bacteria belong to Gluconacetobacter maltaceti.

6. The enzyme preparation according to any one of claims 1 to 5, which is a powdered enzyme preparation.

7. A method of producing the enzyme preparation according to claim 1, the method comprising
culturing the acetic acid bacteria of the Gluconacetobacter in a medium including carbohydrates, vitamins, minerals, and ethanol at an acetic acid acidity of less than 4%, to include as an active ingredient a composite of aldehyde dehydrogenase of the acetic acid bacteria and cell membranes in the culture solution.

8. The method according to claim 7, wherein
culture of the acetic acid bacteria is stopped at the acetic acid acidity of 2 to 3.9%.

9. The method according to claim 7 or 8, wherein
the medium used has an ethanol concentration of 3% or more.

10. The method according to any one of claims 7 to 9, wherein
the acetic acid bacteria used are acetic acid bacteria of the genus Gluconacetobacter, which are capable of growing at the acetic acid acidity of 6% or more.

11. The method according to any one of claims 7 to 10, wherein
the acetic acid bacteria used belong to at least one selected from the group consisting of Gluconacetobacter swingsii, Gluconacetobacter xylinus, Gluconacetobacter europaeus, and Gluconacetobacter maltaceti.

12. An aldehyde removing agent, comprising as an active ingredient the enzyme preparation according to any one of claims 1 to 6.
